(19) Europäisches Patentamt

European Patent Office

Office européen des brevets

(11) Numéro de publication: **0 242 301 B1**

(12) **FASCICULE DE BREVET EUROPEEN**

(45) Date de publication de fascicule du brevet: **28.10.92** (51) Int. Cl.⁵: **A61K 39/10**

(21) Numéro de dépôt: **87400877.4**

(22) Date de dépôt: **16.04.87**

(54) **Procédé de purification d'antigènes protéiques de bactéries appartenant au genre Bordetella, en vue de l'obtention d'un vaccin acellulaire.**

(30) Priorité: **16.04.86 FR 8605456**

(43) Date de publication de la demande:
**21.10.87 Bulletin 87/43**

(45) Mention de la délivrance du brevet:
**28.10.92 Bulletin 92/44**

(84) Etats contractants désignés:
**AT BE CH DE ES GB GR IT LI LU NL SE**

(56) Documents cités:
**EP-A- 0 077 646**
**EP-A- 0 140 386**
**EP-A- 0 162 639**
**FR-A- 2 047 886**

(73) Titulaire: **PASTEUR MERIEUX SERUMS ET VACCINS**
**58, avenue Leclerc**
**F-69007 Lyon(FR)**

(72) Inventeur: **Ouentin-Millet, Marie-José**
**70, Cours Emile Zola**
**F-69100 Villeubanne(FR)**
Inventeur: **Arminjon, François**
**17, Boulevard de la Croix-Rousse**
**F-69004 Lyon(FR)**

(74) Mandataire: **Nony, Michel et al**
**Cabinet NONY & CIE, 29, rue Cambacérès**
**F-75008 Paris(FR)**

## Description

La présente invention a pour objet un perfectionnement au procédé de purification d'antigènes protéiques de bactéries appartenant au genre Bordetella, en vue de l'obtention d'un vaccin acellulaire.

On sait que la culture de bactéries du genre Bordetella, par exemple Bordetella pertussis, Bordetella parapertussis et Bordetella bronchiseptica, permet l'obtention de la protéine F-HA (Filamentous Hemagglutinin) ; de plus la culture de B.pertussis permet également l'obtention d'une exotoxine protéique: la toxine pertussis (encore appelée LPF ou LPF-HA : Leukocytosis Promoting Factor-Hemagglutinin).

Les bactéries du genre Bordetella sont des agents pathogènes connus. On sait en particulier que la bactérie Bordetella pertussis est l'agent pathogène de la coqueluche et que sa culture industrielle est utilisée dans la production de vaccins anticoquelucheux.

On sait également que les antigènes toxine pertussis et F-HA sont susceptibles d'entrer dans la composition d'un vaccin acellulaire anticoquelucheux.

La purification de la toxine pertussis comprend généralement, une étape de purification par chromatographie d'affinité.

L'élution de la toxine pertussis d'un support de chromatographie d'affinité est généralement effectuée à l'aide de tampons renfermant des concentrations élevées de sels et/ou d'agents chaotropiques ou dénaturants tels que le chlorure de magnésium, l'urée, le thiocyanate de sodium ou de potassium, le chlorhydrate de guanidine, etc.. Le principe actif ainsi récupéré en présence de ces produits ne peut pas être utilisé tel quel dans une préparation vaccinale. En conséquence, l'éluat contenant la toxine doit subir une étape supplémentaire ayant pour but l'élimination complète des agents chaotropiques et/ou la réduction des concentrations salines. Cette étape supplémentaire peut consister par exemple en une dialyse exhaustive ou en une filtration sur gel ; voir par exemple le brevet US 4.500.639 et la demande de brevet européen n°0140386.

En outre, l'utilisation de ces techniques d'élution entraîne généralement une insolubilisation partielle et irréversible du principe actif.

La présente invention a pour objet l'utilisation d'une solution tampon particulière qui permet la purification de la toxine pertussis. L'utilisation de ce tampon particulier permet l'élution de la toxine pertussis du support de chromatographie d'affinité. L'utilisation de ce tampon permet d'éluer et donc de purifier en une seule étape la toxine pertussis avec des rendements élevés. L'utilisation de ce même tampon, additionné d'un agent tensio-actif, permet en outre la solubilisation, sans perte, de la toxine pertussis et du F-HA. Ce tampon sert également de solvant dans le procédé de détoxification de la toxine pertussis et permet de maintenir l'anatoxine en solution.

En outre, contrairement aux tampons habituellement utilisés, le tampon particulier de l'invention est compatible avec la préparation d'un vaccin. Autrement dit, les antigènes en solution dans ce tampon peuvent être utilisés directement dans une préparation vaccinale.

La présente invention a donc pour objet l'utilisation, dans la solubilisation et/ou la détoxification de la toxine pertussis et du F-HA des bactéries du genre Bordetella, d'un tampon carbonate ayant un pH de 8,3 à 11,6. Un tel tampon peut être obtenu de façon classique, par exemple en réalisant une solution aqueuse d'un mélange d'un bicarbonate (hydrogénocarbonate) et d'un carbonate alcalin (le métal alcalin étant le sodium ou le potassium) ou encore par mélange en solution aqueuse d'un bicarbonate alcalin et d'un hydroxyde de métal alcalin (soude ou potasse).

De préférence, le tampon carbonate a une molarité de 0,025-0,5M, et un pH de 8,3-11,6.

Le tampon carbonate utilisé selon l'invention peut contenir en outre un agent tensio-actif, par exemple un agent tensio-actif non ionique tel que le Tween 80® (marque commerciale pour le polyoxyéthylène (20) Sorbitan mono oléate).

En règle générale, on utilise de préférence un tampon avec tensio-actif, lorsque cela est nécessaire, pour solubiliser le produit (toxine pertussis ou F-HA) présent sous la forme d'un précipité ou d'un lyophilisat. La concentration du tensio-actif dans le tampon est alors généralement inférieure à 1% en poids, et le plus souvent inférieure à 0,5% en poids. Lorsqu'il s'agit d'éluer la toxine pertussis, on utilise de préférence le tampon carbonate sans tensio-actif.

L'utilisation selon l'invention de tels tampons carbonate présente les avantages qui vont être décrits ci-après.

Le tampon carbonate permet l'élution de la toxine pertussis d'un support de chromatographie d'affinité. Le liquide chromatographié peut être soit un surnageant de culture de bactéries du genre Bordetella, soit une fraction enrichie en toxine pertussis.

On sait que l'utilisation de la toxine pertussis dans un vaccin acellulaire nécessite une opération préalable de détoxification, par exemple par traitement avec le formol ou le glutaraldéhyde. La détoxification a pour but d'éliminer notamment l'effet d'induction de lymphocytose chez la souris, la sensibilisation à l'histamine, l'activité ADP-ribosyl transférase, l'effet cytopathogène sur cellules CHO (Chinese Hamster Ovary), etc....

Une telle détoxification, par exemple à l'aide de formol, conduit à une insolubilisation de la toxine qui rend difficile l'obtention d'une préparation purifiée homogène ; voir par exemple le brevet US 4.455.297.

L'utilisation du tampon carbonate, éventuellement additionné d'un tensio-actif, permet de réaliser l'opération de détoxification en solution et de maintenir en solution l'anatoxine obtenue.

Enfin, comme indiqué précédemment, la solution d'anatoxine pertussis dans le tampon carbonate peut être utilisée directement dans une préparation de vaccin acellulaire.

On a découvert en outre que le tampon carbonate (éventuellement additionné d'un agent tensio-actif, par exemple non ionique, tel que le Tween 80®) favorise la mise en solution du F-HA.

L'invention a également pour objet un procédé de purification des antigènes protéiques produits dans les milieux de culture des bactéries du genre Bordetella, ce procédé étant caractérisé par le fait que l'on met en contact le surnageant de culture, ou une fraction enrichie en toxine pertussis, avec un support solide de chromatographie, capable de fixer la toxine pertussis, puis que l'on élue la toxine dudit support solide à l'aide d'un tampon carbonate ayant un pH de 8,3 à 11,6 et que, si désiré, on soumet l'éluat à une opération de détoxification.

Dans un mode d'exécution particulier, après avoir ajusté le pH à une valeur comprise entre 6 et 8, préférentiellement 7, le surnageant de culture, ou la fraction enrichie en toxine pertussis, est mis en contact avec une glycoprotéine douée d'affinité pour la toxine pertussis, en particulier avec une asialoglycoprotéine (asialofétuine par exemple) couplée à un support chromatographique solide. La quantité de support chromatographique utilisée est fonction du volume de la solution de départ et/ou de la concentration en toxine pertussis dans la fraction à purifier. Le contact se fait par exemple à température de 2 à 30°C, en colonne.

Les glycoprotéines douées d'affinité pour la toxine pertussis sont connues ; on citera par exemple l'haptoglobine, la fétuine, et.... Toutefois, on effectue la chromatographie d'affinité, de préférence, sur ces protéines soumises préalablement à un traitement de désialylation, cette désialylation étant effectuée par hydrolyse acide douce, selon les méthodes connues ; voir par exemple SPIRO et al, J. Biol. Chem. 1974, 249, 5704-5717.

Le couplage de la glycoprotéine ou de la glycoprotéine désialylée peut être effectué selon les méthodes connues. Le support peut être tout support solide classique utilisé habituellement en chromatographie d'affinité.

Le support est notamment un dérivé polyosidique tel qu'un dérivé de cellulose, un dextran réticulé, un gel d'agarose ou le Sépharose-4B, ou bien un support à base de dérivés acryliques tel que le Trisacryl commercialisé par la société IBF (Industrie Biologique Française).

La glycoprotéine peut être fixée sur le support en utilisant par exemple un support activé au CNBr.

Le support peut être également un support de silice poreuse recouverte de DEAE dextran réticulé. On peut utiliser par exemple le Sphérosil recouvert de DEAE dextran.

Le tampon carbonate utilisé dans le procédé de l'invention est de préférence un tampon ayant une molarité de 0,025M à 0,5M et un pH 8,3 à 11,6.

Après élution, la toxine pertussis peut être par exemple précipitée par le sulfate d'ammonium à 50-80% de saturation, ou subir directement une étape de détoxification.

La toxine ainsi purifiée peut être conservée sous la forme d'un précipité au sulfate d'ammonium ou lyophilisée. Le précipité ou le lyophilisat peut être remis en solution dans un tampon carbonate de molarité supérieure à 25mM pH 8,3-11,6 qui peut contenir un détergent tel que le Tween 80® à une concentration finale de l'ordre de 0,05-0,5%, préférentiellement un tampon carbonate 100mM pH 9,6 contenant 0,05% de Tween 80® (appelé ci-après tampon CTW).

Afin d'éliminer le sulfate d'ammonium résiduel ou le support de lyophilisation, la solution est mise en dialyse contre plusieurs volumes du même tampon, à température de 2-30°C, pendant une durée comprise entre 4 et 72 heures. La composition du tampon permettant une solubilisation totale, la solution peut être filtrée sur une membrane de porosité 0,22μm. Cela présente l'avantage de pouvoir procéder aux étapes de détoxification en milieu stérile et à la réalisation d'expérimentations chimiques ou biologiques dont la mise en oeuvre nécessite ou est facilitée par une solubilisation complète du principe actif.

La détoxification éventuelle de la toxine pertussis est effectuée de façon semblable à celle utilisée pour les toxines en général. Selon un autre aspect de la présente invention, le procédé de détoxification est effectué dans un tampon carbonate tel que défini précédemment contenant de préférence un tensio-actif, ce qui favorise à la fois la solubilisation de la toxine et son maintien en solution au cours de la réaction de détoxification. Le rendement de l'étape de détoxification est ainsi proche de 100%. Les agents détoxifiants utilisés sont par exemple le formol ou le glutaraldéhyde.

Après la détoxification, qui peut être effectuée par exemple à une température de 4-40°C, la solution renfermant l'anatoxine peut être dialysée contre le tampon CTW afin d'éliminer toutes traces de l'agent détoxifiant. L'anatoxine ainsi obtenue demeure en solution, elle peut être filtrée sur une membrane stérilisante de porosité 0,22μm, et elle est prête à être incluse dans une préparation vaccinale.

Comme indiqué ci-dessus, le tampon carbonate décrit dans la présente invention permet de plus la solubilisation complète d'une autre protéine isolée des surnageants de culture des bactéries du genre Bordetella : le F-HA. L'utilisation de ce tampon dans l'étape de solubilisation du F-HA présente les avantages décrits dans le cas de la toxine pertussis, c'est-à-dire notamment de pouvoir procéder à une étape de filtration stérilisante sur membrane 0,22μm sans perte de principe actif. Sous cette forme le F-HA peut être directement inclus dans une préparation vaccinale.

Par exemple, le F-HA conservé sous la forme d'un précipité au sulfate d'ammonium est remis en solution selon la technique précédemment décrite pour la toxine pertussis. Le précipité est centrifugé, repris par le tampon CTW et dialysé à température ambiante ou à 4-8°C pendant 4 à 72 heures. Le tampon CTW permet la solubilisation complète du F-HA.

L'invention a également pour objet un vaccin anticoquelucheux acellulaire, caractérisé par le fait qu'il contient au moins un principe actif choisi parmi l'anatoxine pertussis et le F-HA, le ou lesdits principes actifs étant présent(s) en solution dans un tampon carbonate tel que décrit ci-dessus, en particulier dans un tampon carbonate contenant un agent tensio-actif tel que le tampon CTW.

L'anatoxine pertussis et le F-HA en solution dans le tampon carbonate peuvent être directement inclus dans les préparations vaccinales. Celles-ci peuvent contenir soit l'anatoxine seule, soit un mélange d'anatoxine et de F-HA dans les proportions désirées. Afin de corriger le pH final du vaccin à une valeur comprise entre 7 et 8, un certain volume d'eau physiologique tamponnée (tampon PBS) et acidifiée à l'aide d'une solution d'acide concentré est ajouté. Le volume ajouté est fonction du volume et de la molarité du tampon carbonate présent dans la préparation. La concentration en principe(s) actif(s) est alors ajustée à la valeur désirée par addition du volume nécessaire de tampon PBS. A ce stade peuvent être ajoutés au mélange d'autres antigènes (antigènes diphtérique, tétanique, polio, hémophilus, etc...) un agent de conservation tel le merthiolate ou le phénoxyéthanol, et un adjuvant tel qu'un gel d'alumine ou de phosphate de calcium.

Les exemples suivants illustrent l'invention sans toutefois la limiter.

EXEMPLE 1

Purification de toxine pertussis par chromatographie d'affinité.

a) Adsorption de la toxine pertusis sur le support d'affinité

Une fraction enrichie en toxine pertussis obtenue après centrifugation et concentration d'une suspension bactérienne de Bordetella pertussis phase I, cultivée en fermenteur de 30 litres est passée sur une colonne de 4 cm de diamètre contenant 120ml de support de chromatographie Sphépharose 4B couplé à l'asialofétuine, avec un débit de 6 ml/cm$^2$/heure.

Le Sépharose 4-B a été couplé à l'asialofétuine de la façon suivante :

30g de Sépharose-4B activé au CNBr (Pharmacia) sont mis à gonfler dans 6 litres de HCl 1mM pendant 15 minutes environ. Le gel est ensuite lavé 3 fois par 6 litres de HCl 1mM. 400 ml d'une solution contenant 1mg/ml d'asialofétuine, NaHCO$_3$ 0,1 M et NaCl 0,5 M sont ajoutés au gel.

Le mélange est laissé à réagir à + 4°C pendant une nuit sous agitation douce. 125ml d'une solution d'éthanolamine 5 M pH 8,0 sont ajoutés au mélange. Après 4 heures d'incubation à température ambiante, le gel est lavé successivement par 500ml de tampon acétate de sodium 0,1 M pH 4,0 contenant NaCl 1 M puis par 500ml de tampon Tris-HCl 50mM pH 7,5 contenant NaCl 1 M. Ce cycle de lavage est répété trois fois.

Le gel est ensuite lavé trois fois par 500ml de tampon Tris-HCl 50mM pH 7,5 en présence d'un conservateur tel le merthiolate à une concentration de 1/10 000 (p/v).

L'asialofétuine utilisée comme ligand dans la chromatographie d'affinité a été obtenue de la façon suivante :

Une solution aqueuse de fétuine (fétuine type III, Sigma) est hydrolysée par H$_2$SO$_4$ 0,05N pendant 1 heure à 80°C. Après hydrolyse, la solution est dialysée contre plusieurs bains d'eau distillée pendant 24 heures à + 4°C afin d'éliminer les acides sialiques libres. La solution d'asialofétuine peut être concentrée par ultrafiltration à l'aide d'un système équipé de membranes dont le seuil de coupure est égal à 10 000.

L'élimination des acides sialiques est contrôlée par un dosage colorimétrique spécifique des acides sialiques sur la protéine après et avant hydrolyse.

b) Elution de la toxine pertussis

Le gel est lavé avec deux volumes de colonne de tampon Tris-HCl 50mM pH 7,5, c'est-à-dire jusqu'à disparition complète de l'absorption UV à 278 nm, puis avec un volume de colonne de tampon Tris-HCl 50mM pH 7,5 contenant NaCl 1 M. La toxine pertussis est éluée avec 400 ml de tampon carbonate 100 mM pH 9,6.

Le tampon carbonate est préparé comme suit :

On ajoute à 500ml d'une solution de $NaHCO_3$ 0,1 M une solution de $Na_2CO_3$ 0,1 M, jusqu'à un pH final de 9,6 ; il faut ajouter environ 250ml de la solution de $Na_2CO_3$.

Le tampon carbonate ainsi obtenu est filtré sur une membrane de porosité 0,22$\mu$m et conservé à +4°C.

La densité optique et l'activité hémagglutinante des fractions collectées en sortie de colonne sont mesurées.

Les fractions contenant le principe actif, c'est-à-dire celles possédant une forte activité hémagglutinante non inhibée par le cholestérol, sont réunies.

La toxine pertussis est ensuite précipitée par le sulfate d'ammonium à une concentration finale correspondant à 70% de la saturation.

La toxine pertussis ainsi purifiée induit une forte lymphocytose et sensibilise les souris CFW à l'histamine à la dose de 0,04 $\mu$g/souris. La capacité de la toxine d'induire la formation de clusters sur des cellules CHO est caractérisée par une activité spécifique de l'ordre de 65 000 à 260 000 CPU/$\mu$g.

Les résultats de contrôles physico-chimiques et d'activités biologiques (dosages colorimétriques des éventuels contaminants, ADN, ARN, sucre ; dosage du taux d'endotoxine, électrophorèse en milieu SDS ou en milieu acide, etc..) sont en faveur d'une préparation finale homogène renfermant un principe actif hautement purifié.

Les analyses de la teneur en toxine pertussis dans le surnageant de culture initial et dans le précipité final indiquent un rendement de purification supérieur à 90%.

EXEMPLE 2

Etude comparative de la dissolution suivie d'une filtration stérilisante de la toxine pertussis dans différents tampons.

1,2ml d'une suspension de toxine pertussis sous forme d'un précipité au sulfate d'ammonium à 2,6mg/ml sont prélevés et centrifugés 10 minutes à 10 000 x g. Le surnageant est éliminé, le culot est repris par 4ml de tampon CTW et dialysé contre ce même tampon. La solution de toxine est filtrée sur une membrane de porosité 0,22$\mu$m. La concentration en protéine de la solution avant et après filtration est déterminée par un dosage selon la méthode de Lowry et les résultats sont exprimés soit en rendement de filtration : (concentration en toxine après filtration/concentration en toxine avant filtration) x 100, soit en pourcentage relatif calculé par rapport au rendement de filtration dans le tampon CTW auquel on attribue arbitrairement le rendement de 100%.

Les résultats sont présentés dans le tableau ci-après.

TABLEAU I

| Etude comparative des rendements de filtration de toxine pertussis dans différents tampons. | | |
|---|---|---|
| Tampon | Rendement de filtration % | % relatif par rapport au tampon CTW |
| Phosphate de sodium 20mM NaCl 0,5 M pH 7,2 avec 1% Zwittergent TM 3-14* | 50 | 53 |
| Phosphate de sodium 20mM NaCl 0,5 M pH 7,2 avec $\beta$-cyclodextrine $3,5.10^{-6}$ M | 34 | 36 |
| Phosphate de sodium 20mM NaCl 0,5 M pH 7,2 avec 0,05 % Tween 80 | 16 | 17 |
| Phosphate de sodium 20 mM NaCl 0,5 M pH 7,2 avec urée 1 M | 37 | 39 |
| Citrate 100 mM pH 7,0 | 25 | 27 |
| Carbonate 100 mM pH 9,6 | 73 | 78 |
| CTW | 94 | 100 |

* Marque commerciale pour le N-tétradécyl N,N'-diméthyl-3-ammonio-1-propanesulfonate.

EXEMPLE 3 :

Exemple de détoxification de la toxine pertussis.

a) Détoxification par le formol

2ml d'une suspension de toxine pertussis sous forme d'un précipité au sulfate d'ammonium à 5mg/ml (soit 10mg de toxine pertussis) sont centrifugés pendant 15 minutes à 10 000 x g. Le surnageant est éliminé et le culot est repris par 10ml de tampon CTW.

La solution est dialysée une nuit à + 4°C contre 2 litres de tampon CTW. En sortie de dialyse la solution est filtrée sur une membrane de porosité 0,22μm. Un dosage de protéines selon la méthode de Lowry est effectué sur la solution filtrée et la concentration en protéines est amenée à 0,4mg/ml, par addition de tampon CTW. Aux 23ml de solution de toxine à 0,4mg/ml, on ajoute 1,22ml de lysine 1 M en solution dans le tampon CTW et 0,265ml d'une solution de formol à 37%. On laisse incuber par exemple pendant 21 jours à 4°C. Le mélange réactionnel est ensuite dialysé contre le tampon CTW à + 4°C pendant 48 heures afin d'éliminer toute trace de formol. L'anatoxine est ensuite filtrée stérilement sur une membrane de porosité 0,22μm. Sous cette forme l'anatoxine pertussis peut être incorporée dans une préparation vaccinale.

b) Détoxification par le glutaraldéhyde.

15ml d'une solution de toxine pertussis à 500μg/ml dans le tampon CTW sont préparés comme précédemment décrit. 5ml de glutaraldéhyde à 0,10% dans le tampon CTW sont ajoutés à la solution de toxine.

La détoxification peut être conduite à différentes températures, de + 4°C à 40°C ; le temps d'incubation de la toxine en présence de glutaraldéhyde est fonction de la température. Le mélange peut être incubé pendant 48 heures à 4°C par exemple.

Après incubation, 180μl de lysine 1 M en solution dans le tampon CTW sont ajoutés au mélange et la solution est dialysée pendant 48 heures à 4°C contre le tampon CTW. Après dialyse, l'anatoxine pertussis est filtrée sur une membrane de porosité 0,22μm. Sous cette forme, elle peut être incluse dans une préparation vaccinale.

EXEMPLE 4 :

Exemples de solubilisation du F-HA par le tampon carbonate.

Le F-HA cité dans ces exemples a été préparé selon la technique décrite dans la publication de SATO Y, COWELL J.L., SATO H., BURSTYN D.G and MANCLARK C.R. "Separation and Purification of the Hemagglutinins from Bordetella pertussis" Infect. and Immun., 1983, 41, 1, 313,320.

Le F-HA est conservé sous la forme d'un précipité au sulfate d'ammonium à 70% de saturation.

a) 25ml d'une suspension de F-HA à 1,2mg/ml sous la forme d'un précipité au sulfate d'ammonium sont centrifugés 20 minutes à 30 000 x g. Le surnageant est éliminé et le culot est repris par 30ml de tampon CTW.

La solution est dialysée pendant une nuit à + 4°C contre le tampon CTW. La solution est filtrée sur une membrane de porosité 0,22μm. Le F-HA peut être incorporé sous cette forme dans une préparation vaccinale.

b) Une étude comparative des rendements de filtration stérilisante sur une membrane de porosité 0,22μm a été réalisée.

450μl d'une suspension de F-HA précipité au sulfate d'ammonium, à 5,88 mg/ml, sont prélevés et centrifugés 10 minutes à 10 000xg. Le surnageant est éliminé.

Le culot est repris par 3ml de tampon et dialysé contre ce même tampon. La solution de F-HA est filtrée sur une membrane de porosité 0,22μm. La concentration en protéines de la solution avant et après filtration est déterminée par un dosage selon Lowry et les résultats sont exprimés soit en rendements de filtration : (concentration après filtration/concentration avant filtration) x 100, soit en pourcentage relatif calculé par rapport au rendement de filtration dans le tampon CTW auquel on attribue arbitrairement le rendement de 100%.

Les résultats sont présentés dans le tableau II ci-après.

TABLEAU II

| Etude comparative des rendements de filtration du F-HA mis en solution dans différents tampons. | | |
|---|---|---|
| Tampon | Rendement de filtration % | % relatif par rapport au tampon CTW |
| Phosphate d'ammonium 0,1 M pH 4,5 | 54 | 61 |
| Phosphate d'ammonium 0,1 M contenant du NaCl 0,5 M | 3 | 3 |
| Phosphate d'ammonium 0,1 M avec du Tween 80 à 0,05% | 72 | 81 |
| Phosphate monopotassique 0,1 M pH 4,4 | 62 | 70 |
| Phosphate monopotassique 0,1 M contenant NaCl 0,5 M | 2 | 2 |
| Phosphate monopotassique 0,1 M avec du Tween 80 0,05% | 78 | 88 |
| Tris-HCl 50 mM pH 7,4 contenant du NaCL 0,5 M | 58 | 65 |
| CTW | 89 | 100 |

EXEMPLE 5

Préparation d'un vaccin pertussis acellulaire.

Un vaccin pertussis acellulaire renfermant les antigènes purifiés -anatoxine pertussis et F-HA peut être préparé de la façon suivante:

Les deux antigènes en solution dans le tampon CTW sont stérilisés séparément par filtration sur membrane de porosité 0,22 μm et leur concentration déterminée par dosage colorimétrique selon la technique de Lowry par exemple.

Afin de préparer un litre de vaccin pertussis acellulaire renfermant 50μg/ml de chacun des principes actifs, on mélange stérilement les solutions suivantes :

EP 0 242 301 B1

| - Solution d'anatoxine pertussis à 0,38mg/ml dans le tampon CTW | 132 ml |
| - Solution de F-HA à 1,1mg/ml dans le tampon CTW | 46 ml |
| - Eau physiologique tamponnée (PBS) contenant HCl 50mM | 202 ml |
| - Hydroxyde d'aluminium à 10mg d'Al/ml | 20 ml |
| - Merthiolate 1% PBS (P/V) | 10 ml |
| - PBS   qsp | 1 l |

Cette préparation vaccinale présente les caractéristiques suivantes:

| - Anatoxine pertussis | 50 $\mu$g/ml |
| - F-HA | 50 $\mu$g/ml |
| - Aluminium | 200 $\mu$g/ml |
| - pH | 7,6 |
| - Osmolalité | 265 mosm/kg |
| - Merthiolate | 1/10.000 |

**Revendications**

**Revendications pour les Etats contractants suivants : AT, BE, CH, DE, GB, GR, IT, LI, LU NL, SE**

1. Utilisation dans la solubilisation et/ou la détoxification des antigènes protéiques des bactéries du genre Bordetella, ou dans l'élution de la toxine pertussis, d'un tampon carbonate ayant un pH de 8,3 à 11,6.

2. Utilisation selon la revendication 1, caractérisée par le fait que ledit tampon carbonate est un mélange d'un bicarbonate et d'un carbonate alcalin.

3. Utilisation selon l'une quelconque des revendications précédentes, caractérisée par le fait que le tampon carbonate a une molarité de 0,025-0,5 M.

4. Utilisation selon l'une quelconque des revendications précédentes, caractérisée par le fait que ledit tampon contient en outre un agent tensio-actif, à une concentration inférieure à 1% en poids.

5. Procédé de purification des antigènes protéiques produits dans les milieux de culture des bactéries du genre Bordetella, caractérisé par le fait que l'on met en contact le surnageant de culture, ou une fraction enrichie en toxine pertussis, avec un support solide de chromatographie, capable de fixer la toxine pertussis, puis que l'on élue la toxine dudit support solide à l'aide d'un tampon carbonate ayant un pH de 8,3 à 11,6 et que, si désiré, on soumet l'éluat à une opération de détoxification.

6. Procédé selon la revendication 5, caractérisé par le fait que le tampon carbonate a une molarité de 0,025M à 0,5M.

7. Procédé de solubilisation d'antigènes protéiques produits dans les milieux de culture des bactéries du genre Bordetella, caractérisé par le fait que l'on dissout lesdits antigènes, sous forme d'un précipité ou d'un lyophilisat, dans un tampon carbonate de pH 8,3-11,6 additionné d'un tensio-actif à une concentration inférieure à 1% en poids.

8. Procédé de détoxification de la toxine pertussis, à l'aide d'un agent de détoxification usuel, caractérisé par le fait que l'on effectue la détoxification dans un tampon carbonate tel que défini dans l'une quelconque des revendications 1 à 4.

9. Vaccin anticoquelucheux acellulaire, caractérisé par le fait qu'il contient au moins un principe actif choisi parmi l'anatoxine pertussis et le F-HA, le ou lesdits principes actifs étant présent(s) en solution dans un tampon carbonate tel que défini dans l'une quelconque des revendications 1 à 4.

**Revendications pour l'Etat contractant suivant : ES**

8

1. Procédé de solubilisation ou de détoxification des antigènes protéiques des bactéries du genre Bordetella, ou d'élution de la toxine pertussis, dans lequel on effectue ces opérations à l'aide d'un tampon carbonate ayant un pH de 8,3 à 11,6.

2. Procédé selon la revendication 1, caractérisée par le fait que ledit tampon carbonate est un mélange d'un bicarbonate et d'un carbonate alcalin.

3. Procédé selon l'une quelconque des revendications précédentes, caractérisée par le fait que le tampon carbonate a une molarité de 0,025-0,5 M.

4. Procédé de purification des antigènes protéiques produits dans les milieux de culture des bactéries du genre Bordetella, caractérisé par le fait que l'on met en contact le surnageant de culture, ou une fraction enrichie en toxine pertussis, avec un support solide de chromatographie, capable de fixer la toxine pertussis, puis que l'on élue la toxine dudit support solide à l'aide d'un tanpon carbonate ayant un pH de 8,3 à 11,6 et que, si désiré, on soumet l'éluat à une opération de détoxification.

5. Procédé selon la revendication 4, caractérisé par le fait que le tampon carbonate a une molarité de 0,025M à 0,5M.

6. Procédé de solubilisation d'antigènes protéiques produits dans les milieux de culture des bactéries du genre Bordetella, caractérisé par le fait que l'on dissout lesdits antigènes, sous forme d'un précipité ou d'un lyophilisat, dans un tampon carbonate de pH 8,3-11,6 additionné d'un tensio-actif à une concentration inférieure à 1% en poids.

7. Procédé de détoxification de la toxine pertussis, à l'aide d'un agent de détoxification usuel, caractérisé par le fait que l'on effectue la détoxification dans un tampon carbonate tel que défini dans l'une quelconque des revendications 1 à 3.

8. Procédé selon la revendication 7, caractérisé par le fait que ledit tampon contient en outre un agent tensio-actif, à une concentration inférieure à 1% en poids.

**Claims**
**Claims for the following Contracting States : AT, BE, CH, DE, GB, GR, IT, LI, LU, NL, SE**

1. Use of a carbonate buffer having a pH of 8.3 to 11.6 in the Solubilization and/or detoxification of the protein antigens of bacteria of the genus Bordetella, or in the elution of the pertussis toxin.

2. Use according to Claim 1, characterized in that the said carbonate buffer is a mixture of a bicarbonate and an alkali metal carbonate.

3. Use according to either of the preceding claims, characterized in that the carbonate buffer has a molarity of 0.025-0.5 M.

4. Use according to any one of the preceding claims, characterized in that the said buffer contains, in addition, a surfactant, at a concentration of less than 1% by weight.

5. Process for purifying the protein antigens produced in the culture media of bacteria of the genus Bordetella, characterized in that the culture supernatent, or a fraction enriched with respect to pertussis toxin, is brought into contact with a solid chromatography support capable of binding the pertussis toxin, in that the toxin is then eluted from the said solid support by means of a carbonate buffer having a pH of 8.3 to 11.6, and in that, if desired, the eluate is subjected to a detoxification operation.

6. Process according to Claim 5, characterized in that the carbonate buffer has a molarity of 0.025 M to 0.5 M.

7. Process for solubilizing protein antigens produced in the culture media of bacteria of the genus Bordetella, characterized in that the said antigens, in the form of a precipitate or a lyophilizate, are dissolved in a carbonate buffer having a pH of 8.3-11.6 to which a surfactant has been added at a concentration of less than 1% by weight.

**8.** Process for detoxifying pertussis toxin by means of a customary detoxifying agent, characterized in that the detoxification is performed in a carbonate buffer as defined in any one of Claims 1 to 4.

**9.** Acellular anti-whooping cough vaccine, characterized in that it contains at least one active principle chosen from pertussis anatoxin and the F-HA, the said active principle or principles being present in solution in a carbonate buffer as defined in any one of Claims 1 to 4.

**Claims for the following Contracting State : ES**

**1.** Process for solubilizing or detoxifying the protein antigens of bacteria of the genus Bordetella, or for eluating the pertussis toxin, wherein these steps are performed with the help of a carbonate buffer having a pH of 8.3 to 11.6

**2.** Process according to Claim 1, characterized in that the said carbonate buffer is a mixture of a bicarbonate and an alkali metal carbonate.

**3.** Process according to either of the preceding claims, characterized in that the carbonate buffer has a molarity of 0.025-0.5 M.

**4.** Process for purifying the protein antigens produced in the culture media of bacteria of the genus. Bordetella, characterized in that the culture supernatent, or a fraction enriched with respect to pertussis toxin, is brought into contact with a solid chromatography support capable of binding the pertussis toxin, in that the toxin is then eluted from the said solid support by means of a carbonate buffer having a pH of 8.3 to 11.6, and in that, if desired, the eluate is subjected to a detoxification operation.

**5.** Process according to Claim 4, characterized in that the carbonate buffer has a molarity of 0.025 M to 0.5 M.

**6.** Process for solubilizing protein antigens produced in the culture media of bacteria of the genus Bordetella, characterized in that the said antigens, in the form of a precipitate or a lyophilizate, are dissolved in a carbonate buffer having a pH of 8.3-11.6 to which a surfactant has been added at a concentration of less than 1% by weight.

**7.** Process for detoxifying pertussis toxin by means of a customary detoxifying agent, characterized in that the detoxification is performed in a carbonate buffer as defined in any one of Claims 1 to 3

**8.** Process according to claim 7, characterized in that the said buffer contains, in addition, a surfactant, at a concentration of less than 1% by weight.

**Patentansprüche**
**Patentansprüche für folgende Vertragsstaaten : AT, BE, CH, DE, GB, GR, IT, LI, LU, NL, SE**

**1.** Verwendung eines Carbonatpuffers mit einem pH-Wert 8,3 bis 11,6 zur Solubilisation und/oder Entgiftung von proteinischen Antigenen von Bakterien des Stanmes Bortella, oder zur Eluierung von Keuchhusten-Toxin.

**2.** Verwendung nach Anspruch 1, **dadurch gekennzeichnet,** dass der Carbonatpuffer ein Gemisch aus einem Bicarbonat und einem Carbonat eines Alkalimetalls ist.

**3.** Verwendung nach einem der vorhergehenden Ansprüchen **dadurch gekennzeichnet,** dass der Carbonatpuffer eine Molarität von 0,025 bis 0,5 hat.

**4.** Verwendung nach einem der vorhergehenden Ansprüche, **dadurch gekennzeichnet,** dass der Puffer weiterhin ein oberflächenaktives Mittel in einer Konzentration unter 1 Gew.% enthält.

**5.** Verfahren zur Reinigung von proteinischen Antigenen, die in einem Kulturmilieu von Bakterien des Stammes Bordetella erzeugt worden sind, **dadurch gekennzeichnet,** dass man die überstehende Flüssigkeit der Kultur oder eine Fraktion, die mit Keuchhusten-Toxin angereichert ist, mit einem festen chromatographischen Trägermaterial in Berührung bringt, das in der Lage ist, das Keuchhusten-Toxin zu fixieren, und dass man das auf dem festen Trägermaterial befindliche Toxin mittels eines Carbonatpuffers von pH-Wert 8,3 bis 11,6 eluiert und gegebenenfalls das Eluat einer Entgiftungsbehandlung unterzieht.

6. Verfahren nach Anspruch 5, **dadurch gekennzeichnet,** dass der Carbonatpuffer eine Molarität von 0,025 bis 0,5 hat.

7. Verfahren zur Solubilisierung von proteinischen Antigenen, die in einem Kulturmilieu von Bakterien des Stamms Bordetella erzeugt worden sind, **dadurch gekennzeichnet,** dass man die Antigene in Lösung bringt unter Bildung eines Niederschlags oder eines Lyophilisats in einem Carbonatpuffer von pH-Wert 8,3 bis 11,6 und unter Zusatz eines oberflächenaktiven Mittels einer Konzentration unter 1 Gew.%.

8. Verfahren zur Entgiftung von Keuchhusten-Toxin mittels eines üblichen Entgiftungsmittels, **dadurch gekennzeichnet,** dass man die Entgiftung in einem Carbonatpuffer durchführt, wie er in einem der Ansprüche 1 bis 4 definiert ist.

9. Zellfreie Vaccine gegen Keuchhusten, **dadurch gekennzeichnet,** dass sie mindestens einen Wirkstoff enthält, ausgewählt aus Keuchhusten-Anatoxin und F-HA, wobei einer dieser Wirkstoffe in Lösung in einem Carbonatpuffer vorliegt, wie er definiert ist in einem der Ansprüche 1 bis 4.

**Patentansprüche für folgenden Vertragsstaat : ES**

1. Verfahren zur Solubilisierung oder Entgiftung von proteinischen Antigenen von Bakterien des Stammes Bordetella oder zur Eluierung von Keuchhusten- Toxin, wohin diese Stufe mit Hilfe eines Carbonatpuffers mit einem pH-wert 8,3 bis 11,6 durchgeführt sind.

2. Verfahren nach Anspruch 1, **dadurch gekennzeichnet,** dass der Carbonatpuffer ein Gemisch aus einem Bicarbonat und einem Carbonat eines Alkalimetalls ist.

3. Verfahren nach einem der vorhergehenden Ansprüche, **dadurch gekennzeichnet,** dass der Carbonatpuffer eine Molarität von 0,025 bis 0,5 hat.

4. Verfahren zur Reinigung von proteinischen Antigenen, die in einem Kulturmilieu von Bakterien des Stammes Bordetella erzeugt worden sind, **dadurch gekennzeichnet,** dass man die überstehende Flüssigkeit der Kultur oder eine Fraktion, die mit Keuchhusten-Toxin angereichert ist, mit einem festen chromatographischen Trägermaterial in Berührung bringt, das in der Lage ist, das Keuchhusten-Toxin zu fixieren, und dass man das auf dem festen Trägermaterial befindliche Toxin mittels eines Carbonatpuffers von pH-Wert 8,3 bis 11,6 eluiert und gegebenenfalls das Eluat einer Entgiftungsbehandlung unterzieht.

5. Verfahren nach Anspruch 4 **dadurch gekennzeichnet,** dass der Carbonatpuffer eine Molarität von 0,025 bis 0,5 hat.

6. Verfahren zur Solubilisierung von proteinischen Antigenen, die in einem Kulturmilieu von Bakterien des Stamms Bordetella erzeugt worden sind, **dadurch gekennzeichnet,** dass man die Antigene in Lösung bringt unter Bildung eines Niederschlags oder eines Lyophilisats in einem Carbonatpuffer von pH-Wert 8,3 bis 11,6 und unter Zusatz eines oberflächenaktiven Mittels einer Konzentration unter 1 Gew.%.

7. Verfahren zur Entgiftung von Keuchhusten-Toxin mittels eines üblichen Entgiftungsmittels, **dadurch gekennzeichnet,** dass man die Entgiftung in einem Carbonatpuffer durchführt, wie er in einem der Ansprüche 1 bis 3 definiert ist.

8. Verfahren nach Anspruch 7, dardurch gekennzeichnet, dass der Puffer weiterhin ein oberflächenaktives Mittel in einer Konzentration unter 1 Gew.% enthält.